# EUROPEAN PATENT APPLICATION

(11) **EP 4 378 934 A1**
(43) Date of publication of application: **05.06.2024**
(21) Application number: 22849182.5
(22) Date of filing: 05.07.2022
(51) Int. Cl.: C07D 317/36, C07D 317/38, C07D 317/50, B01D 53/02, B01D 53/75, B01D 53/81

(54) **RECOVERY METHOD AND PRODUCTION METHOD FOR CYCLIC CARBONATE**

(30) Priority: 26.07.2021 JP 2021121573
(71) Applicant: Sumitomo Seika Chemicals Co., Ltd., Kako-gun, Hyogo 675-0145 (JP)
(72) Inventor: HAYAMIZU, Naohisa, Himeji-shi, Hyogo 672-8076 (JP)
(74) Representative: Vossius & Partner Patentanwälte Rechtsanwälte mbB
(86) International application number: PCT/JP2022/026754
(87) International publication number: WO 2023/008117

(57) **Abstract**

Provided is a method for effectively using a polyalkylene carbonate as an organic binder in the production of ceramic components. Specifically, provided is a method for recovering or producing a cyclic carbonate, comprising recovering a cyclic carbonate contained in a decomposition gas generated by thermal decomposition of a polyalkylene carbonate.

## Description

### Technical Field

The present disclosure relates to, for example, a recovery method and production method for a cyclic carbonate, which is a decomposition product of polycarbonate. The contents of all documents listed in the present specification are incorporated herein by reference.

### Background Art

Ceramic products are widely used in various fields, such as insulating materials, heat-resistant materials, and mechanical parts. These materials are produced by mixing a starting material powder for ceramics with an organic binder, and subjecting the mixture to molding using various methods, such as injection molding, extrusion molding, casting, and tape molding, followed by dewaxing and sintering steps. Among ceramic products, multilayer ceramic capacitors (MLCCs), for example, are in high demand and are actively produced by this method.

As the organic binder, typically, methylcellulose, polyvinyl alcohol, polyvinyl butyral, acrylic resin, and the like have been used. Additionally, as the organic binder, the potentiality of use of a polyalkylene carbonate has also been suggested (e.g., Patent Literature (PTL) 4, PTL 5, and PTL 6).

### Citation List

### Patent Literature

PTL 1: JP2001-213669A
PTL 2: JP2004-67447A
PTL 3: JP2002-260952A
PTL 4: JP2011-020916A
PTL 5: JP2014-055232A
PTL 6: WO2020/153329

### Summary of Invention

### Technical Problem

The present inventor has studied whether a polyalkylene carbonate can be effectively used as an organic binder in the production of ceramic components.

### Solution to Problem

The present inventor confirmed that the use of a polyalkylene carbonate as an organic binder in the production of ceramics is possible. Furthermore, in the course of the study, the inventor noticed that the gas generated by heating and decomposing a polyalkylene carbonate for dewaxing contains a cyclic carbonate. The generated cyclic carbonate is preferably recovered. Further, cyclic carbonates are highly valuable materials that can be used as a starting material for electrolytes and polycarbonates. Thus, the inventor conducted further study on the recovery of a cyclic carbonate from the polyalkylene carbonate that was used as a binder in the production of ceramics.

The present disclosure encompasses, for example, the subject matter described in the following items.

### Item 1.

A method for recovering a cyclic carbonate, comprising:
(B) recovering a cyclic carbonate contained in a decomposition gas generated by thermal decomposition of a polyalkylene carbonate.

### Item 2.

The method according to Item 1, further comprising, before (B):
(A) thermally decomposing and gasifying a polyalkylene carbonate.

### Item 3.

The method according to Item 1 or 2, wherein the recovery of the decomposition gas is performed after concentrating the decomposition gas.

### Item 4.

The method according to Item 2, comprising:
(a) heating a mixture containing a polyalkylene carbonate and an inorganic material to decompose and gasify the polyalkylene carbonate; and
(b) concentrating and recovering the obtained gas.

### Item 5.

The method according to Item 3 or 4, wherein the gas concentration is performed by at least one operation selected from the group consisting of the following (i), (ii), and (iii):
(i) causing a polyalkylene carbonate decomposition gas to be adsorbed to a gas adsorbent;
(ii) separating a polyalkylene carbonate decomposition gas with a gas separation material; and
(iii) cooling and liquefying a polyalkylene carbonate decomposition gas.

### Item 6.

The method according to any one of Items 1 to 5, wherein the cyclic carbonate is at least one member selected from the group consisting of ethylene carbonate, propylene carbonate, butylene carbonate, and cyclohexene carbonate.

### Item 7.

A method for producing a cyclic carbonate, comprising:
(B) recovering a cyclic carbonate contained in a decomposition gas generated by thermal decomposition of a polyalkylene carbonate.

### Item 8.

The method according to Item 7, further comprising, before (B):
(A) thermally decomposing and gasifying a polyalkylene carbonate.

### Item 9.

The method according to Item 7 or 8, wherein the recovery of the decomposition gas is performed after concentrating the decomposition gas.

### Item 10.

The method according to Item 8, comprising:
(a) heating a mixture containing a polyalkylene carbonate and an inorganic material to decompose and gasify the polyalkylene carbonate, and
(b) concentrating and recovering the obtained gas.

### Item 11.

The method according to Item 9 or 10, wherein the gas concentration is performed by at least one operation selected from the group consisting of the following (i), (ii), and (iii):
(i) causing a polyalkylene carbonate decomposition gas to be adsorbed to a gas adsorbent;
(ii) separating a polyalkylene carbonate decomposition gas with a gas separation material; and
(iii) cooling and liquefying a polyalkylene carbonate decomposition gas.

### Item 12.

The method according to any one of Items 7 to 11, wherein the cyclic carbonate is at least one member selected from the group consisting of ethylene carbonate, propylene carbonate, butylene carbonate, and cyclohexene carbonate.

### Advantageous Effects of Invention

Provided is a method for recovering or producing a cyclic carbonate from a gas obtained by thermally decomposing a polyalkylene carbonate that has been used.

### Description of Embodiments

Embodiments encompassed by the present disclosure are described in more detail below. The present disclosure encompasses, for example, a method for recovering or producing a cyclic carbonate.

The method for recovering or producing a cyclic carbonate encompassed by the present disclosure comprises recovering a cyclic carbonate contained in a decomposition gas generated by thermal decomposition of a polyalkylene carbonate. This method for recovering or producing a cyclic carbonate may be referred to as "the method of the present disclosure."

The polyalkylene carbonate for use in the method of the present disclosure is obtained, for example, by copolymerization of an epoxide and carbon dioxide. Examples of epoxides include, but are not limited to, ethylene oxide, propylene oxide, 1-butene oxide, 2-butene oxide, isobutylene oxide, 1-pentene oxide, 2-pentene oxide, 1-hexene oxide, 1-octene oxide, 1-decene oxide, cyclopentene oxide, cyclohexene oxide, styrene oxide, vinylcyclohexane oxide, 3-phenylpropylene oxide, allyl glycidyl ether, methyl glycidyl ether, phenyl glycidyl ether, and epichlorohydrin. Of these, ethylene oxide, propylene oxide, 1-butene oxide, and cyclohexene oxide are preferred. These epoxides may be used alone or in a combination of two or more. When the epoxide contains ethylene oxide, the obtained polycarbonate contains polyethylene carbonate. When the epoxide contains propylene oxide, the obtained polycarbonate contains polypropylene carbonate. When the epoxide contains 1-butene oxide, the obtained polycarbonate contains polybutylene carbonate. Further, when the epoxide contains cyclohexene oxide, the obtained polycarbonate contains polycyclohexene carbonate.

When two or more epoxides are combined, the obtained polycarbonate can be a copolymer. For example, when propylene oxide and cyclohexene oxide are used as epoxides, the obtained polycarbonate can be poly(propylene/cyclohexene) carbonate, which is a copolymer. The obtained copolymer may be a random copolymer or a block copolymer. More preferred is a random copolymer.

The polyalkylene carbonates may be used alone or in a combination of two or more.

The heating temperature for thermal decomposition of polyalkylene carbonate is not limited as long as the temperature is within the range in which a polyalkylene carbonate is decomposed and gasified. For example, the temperature may be 100 to 800°C. The upper limit or the lower limit of the range may be, for example, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, or 790°C. For example, the range may be 150 to 750°C.

As stated above, the method of the present disclosure is preferably applicable when a polyalkylene carbonate is used as a binder in the production of ceramic components and is dewaxed by thermal decomposition. The dewaxing step is usually performed in a heating apparatus (e.g., a furnace) while allowing a less-reactive gas, such as nitrogen gas or argon gas, to flow into the apparatus. This is to allow the gas generated by the dewaxing step (heating) to be discharged out of the apparatus. The exhaust gas generated by the dewaxing step becomes a mixed gas of a polyalkylene carbonate decomposition gas and the inflow gas. For example, when such a gas obtained by thermal decomposition of polyalkylene carbonate contains a small amount of a polyalkylene carbonate decomposition gas (or when the polyalkylene carbonate decomposition gas is diluted), it is preferable to recover the polyalkylene carbonate decomposition gas by first concentrating the polyalkylene carbonate decomposition gas from the obtained gas (the resulting gas).

Examples of the concentration means (concentration operations) include the following (i), (ii), and (iii). These means (i) to (iii) may be used alone or in a combination of two or three.
(i) Causing a polyalkylene carbonate decomposition gas to be adsorbed to a gas adsorbent,
(ii) separating a polyalkylene carbonate decomposition gas with a gas separation device, and
(iii) cooling and liquefying a polyalkylene carbonate decomposition gas.

By bringing the resulting gas into contact with a gas adsorbent, it is possible to (i) cause a polyalkylene carbonate decomposition gas to be adsorbed to a gas adsorbent. Examples of the gas adsorbent include activated carbon, silica gel, zeolite, and activated alumina. Gas adsorbents may be used alone or in a combination of two or more.

Further, the resulting gas can be separated into a polyalkylene carbonate decomposition gas and other gases (e.g., the ambient gases mentioned above) with a gas separation device. That is, it is possible to (ii) separate a polyalkylene carbonate decomposition gas with a gas separation device. Examples of the gas separation device include a gas separation membrane and a pressure swing adsorption (PSA) device.

Further, by cooling the resulting gas while taking into account the boiling point of each gas contained in the resulting gas, a liquid in which a polyalkylene carbonate decomposition gas is concentrated can be obtained. That is, it is possible to (iii) cool and liquefy a polyalkylene carbonate decomposition gas. For example, by performing (iii) above after concentrating the polyalkylene carbonate decomposition gas according to (i) and/or (ii) above, a liquid in which a polyalkylene carbonate decomposition gas is concentrated can be more efficiently obtained.

These operations can be performed, for example, using known apparatuses. For example, when a polyalkylene carbonate is used as a binder in the production of ceramics and is dewaxed by thermal decomposition, the generated exhaust gas is treated by using a condensing or cooling apparatus for exhaust gas. The apparatuses used here may be such known apparatuses. In particular, a condensing apparatus can be preferably used for (i) and/or (ii) above, and a cooling apparatus can be preferably used for (iii) above.

The cooling temperature is preferably equal to or below the temperature at which the cyclic carbonate contained in the polyalkylene carbonate decomposition gas can be liquefied. The temperature is preferably lower than the boiling point of a cyclic carbonate, which is a decomposition product of polyalkylene carbonate, and is preferably, for example, 5 to 50°C, 5 to 40°C, 5 to 30°C, 10 to 50°C, 10 to 40°C, or 10 to 30°C.

The method of the present disclosure enables efficient recovery or production of a cyclic carbonate. The type of the polyalkylene carbonate to be decomposed determines the type of the cyclic carbonate recovered or produced. A polyalkylene carbonate, which is obtained by copolymerization of an epoxide and carbon dioxide, produces a cyclic carbonate whose single molecule consists of a single molecule of the epoxide and a single molecule of carbon dioxide. For example, if the polyalkylene carbonate for use is a copolymer of ethylene oxide and carbon dioxide, ethylene carbonate is produced. If it is a copolymer of propylene oxide and carbon dioxide, propylene carbonate is produced. If it is a copolymer of 1-butene oxide and carbon dioxide, butylene carbonate is produced. If it is a copolymer of cyclohexene oxide and carbon dioxide, cyclohexene carbonate is produced.

As stated above, polyalkylene carbonates may be used alone or in a combination of two or more. If a single type of polyalkylene carbonate is used, a single type of cyclic carbonate is produced. If two or more types of polyalkylene carbonate are used, two or more types of cyclic carbonate are usually produced.

As stated above, the method for recovering or producing a cyclic carbonate described above is preferably applicable, for example, when a polyalkylene carbonate is used as a binder in the production of ceramic components and is dewaxed by thermal decomposition. In the production of ceramics, a polyalkylene carbonate is used, for example, by mixing with an inorganic material, which is a starting material for ceramics. The method of the present disclosure can be preferably performed by heating the mixture and using the obtained polyalkylene carbonate decomposition gas. Polyalkylene carbonates can be preferably used not only for producing ceramics but also as a composition for dispersing an inorganic material. Thus, the scope of application of the method of the present disclosure is not limited to the production of ceramic components.

Examples of the inorganic material used in combination with a polyalkylene carbonate include ceramics, conductor powders, glass powders, and phosphor particles, depending on the purpose and use thereof. These may be used alone or in a combination of two or more.

Examples of ceramics include aluminum oxide, zirconium oxide, titanium oxide, barium titanate, strontium titanate, zirconium titanate, lead zirconate titanate, lanthanum vanadate, ferrite, zinc oxide, magnesium oxide, beryllium oxide, aluminum nitride, silicon nitride, boron nitride, gallium nitride, silicon carbide, zirconium carbide, magnesium fluoride, tin-doped indium oxide, antimony-doped tin oxide, aluminum-doped zinc oxide, and other ceramics.

Examples of conductor powders include metals, such as copper, iron, nickel, palladium, platinum, gold, silver, aluminum, tungsten, and tin, alloys thereof, and carbon materials, such as graphite, carbon black, and carbon nanotubes.

Examples of glass powders include glass powders of various silicon oxides, such as the CaO-Al₂O₃-SiO₂ system, MgO-Al₂O₃-SiO₂ system, and LiO₂-Al₂O₃-SiO₂ system, bismuth oxide glass, silicate glass, lead glass, zinc glass, and boron glass.

Examples of phosphor particles include Y₂SiO₅:Ce, CaWO₄:Pb, BaMgAl₁₄O₂₃:Eu, Y₂O₃:Eu, Y₂SiO₅:Eu, Y₃A₁₅O₁₂:Eu, Zn₃(PO₄)₂:Mn, YBO₃:Eu, GdBO₃:Eu, ScBO₃:Eu, LuBO₃:Eu, Zn₂SiO₄:Mn, BaAl₁₂O₁₉:Mn, CaAl₁₂O₁₉:Mn, YBO₃:Tb, BaMgAl₁₄O₂₃:Mn, LuBO₃:Tb, and BaMgAl₁₂O₂₃: Eu.

The inorganic material is preferably in a powder form. In this case, the size of the inorganic particles is not particularly limited. From the viewpoint of obtaining a dense molded body, it is preferable to use those having a median diameter as determined by a laser diffraction/scattering method of 0.01 to 20 µm.

The conditions under which a polyalkylene carbonate as a binder is thermally decomposed in a dewaxing step etc. are modified according to the type of ceramics and the field of the product. For example, for multilayer ceramic capacitors (MLCCs), thermal decomposition is performed in a nitrogen atmosphere. Since cyclic carbonates decompose into water and carbon dioxide in the presence of oxygen, thermal decomposition is preferably performed in the absence of oxygen to increase the recovery of cyclic carbonate.

According to the method of the present disclosure, a cyclic carbonate can be recovered or produced efficiently. In particular, the method enables recovery of, as a cyclic carbonate, a significant amount of the polyalkylene carbonate used. Preferably, the recovery can be made such that the conversion of the polyalkylene carbonate used into a cyclic polycarbonate is 90 mass% or more, more preferably 91, 92, 93, 94, or 95 mass% or more, and still more preferably 96, 97, 98, or 99 mass% or more.

Recovering useful substances, such as cyclic carbonates, with high efficiency from a gas after dewaxing of, for example, organic binders that have been used in the production of ceramics (e.g., polyvinyl butyral) is thought to be difficult. Thus, the method of the present disclosure increases the superiority of using a polyalkylene carbonate as an organic binder.

In addition, it is thought to be typical for a polyalkylene carbonate decomposition gas to be treated by combustion. In other words, in the past, a polyalkylene carbonate decomposition gas was considered an unnecessary product that could be easily treated, and the need for a novel treatment method also did not exist. According to the method of the present disclosure, a polyalkylene carbonate decomposition gas can be used as a starting material for producing a cyclic carbonate, which is a useful substance; this point is considered to be another advantage of the method of the present disclosure.

In the present specification, the terms "comprising" and "containing" also include "consisting essentially of" and "consisting of." The present disclosure encompasses any combination of the elements described in the present specification.

In addition, the various characteristics (properties, structures, functions, etc.) described in each embodiment of the present disclosure described above may be combined in any way in specifying the subjects encompassed by the present disclosure. In other words, the present disclosure encompasses all the subjects comprising all combinations of the combinable characteristics described in the present specification.

### Examples

The embodiments of the present disclosure are described with reference to examples in more detail below. However, the embodiments of the present disclosure are not limited to the following Examples.

### Study on Recovery of Cyclic Carbonate from Polyalkylene Carbonate Decomposition Gas

Polypropylene carbonate was heated at each temperature listed in Table 1, and the generated decomposition gas was analyzed by gas chromatograph (GC) to determine the amount of propylene carbonate contained in the decomposition gas (specifically, a calibration curve was prepared for quantitative analysis). For GC, GC-2010 (Shimadzu Corporation) (column: DB-17, detector: FID) was used. For a device for thermal decomposition, JHP-5 (Japan Analytical Industry Co., Ltd.) was used.

The ratio (%) of the mass of propylene carbonate in the decomposition gas to the mass of the thermally decomposed polypropylene carbonate was calculated as the "PC conversion." Table 1 also shows the results thereof. (Values exceeding 100% are believed to be errors.)

**Table 1**

| Decomposition temperature | PC conversion |
|---|---|
| (°C) | (%) |
| 315 | 99 |
| 358 | 99 |
| 386 | 101 |
| 423 | 103 |

The gas obtained by decomposing polyalkylene carbonate was confirmed to comprise a cyclic carbonate that is almost 100% derived from the polyalkylene carbonate.

## Claims

1. A method for recovering a cyclic carbonate, comprising:
(B) recovering a cyclic carbonate contained in a decomposition gas generated by thermal decomposition of a polyalkylene carbonate.

2. The method according to claim 1, further comprising, before (B):
(A) thermally decomposing and gasifying a polyalkylene carbonate.

3. The method according to claim 1 or 2, wherein the recovery of the decomposition gas is performed after concentrating the decomposition gas.

4. The method according to claim 2, comprising:
(a) heating a mixture containing a polyalkylene carbonate and an inorganic material to decompose and gasify the polyalkylene carbonate; and
(b) concentrating and recovering the obtained gas.

5. The method according to claim 3, wherein the gas concentration is performed by at least one operation selected from the group consisting of the following (i), (ii), and (iii):
(i) causing a polyalkylene carbonate decomposition gas to be adsorbed to a gas adsorbent;
(ii) separating a polyalkylene carbonate decomposition gas with a gas separation material; and
(iii) cooling and liquefying a polyalkylene carbonate decomposition gas.

6. The method according to claim 1 or 2, wherein the cyclic carbonate is at least one member selected from the group consisting of ethylene carbonate, propylene carbonate, butylene carbonate, and cyclohexene carbonate.

7. A method for producing a cyclic carbonate, comprising:
(B) recovering a cyclic carbonate contained in a decomposition gas generated by thermal decomposition of a polyalkylene carbonate.

8. The method according to claim 7, further comprising, before (B):
(A) thermally decomposing and gasifying a polyalkylene carbonate.

9. The method according to claim 7 or 8, wherein the recovery of the decomposition gas is performed after concentrating the decomposition gas.

10. The method according to claim 8, comprising:
(a) heating a mixture containing a polyalkylene carbonate and an inorganic material to decompose and gasify the polyalkylene carbonate, and
(b) concentrating and recovering the obtained gas.

11. The method according to claim 9, wherein the gas concentration is performed by at least one operation selected from the group consisting of the following (i), (ii), and (iii):
(i) causing a polyalkylene carbonate decomposition gas to be adsorbed to a gas adsorbent;
(ii) separating a polyalkylene carbonate decomposition gas with a gas separation material; and
(iii) cooling and liquefying a polyalkylene carbonate decomposition gas.

12. The method according to claim 7 or 8, wherein the cyclic carbonate is at least one member selected from the group consisting of ethylene carbonate, propylene carbonate, butylene carbonate, and cyclohexene carbonate.
